# EUROPEAN PATENT SPECIFICATION

(11) **EP 0 542 964 B1**
(45) Date of publication and mention of the grant of the patent: **13.09.1995**
(21) Application number: 92911560.8
(22) Date of filing: 04.06.1992
(51) Int. Cl.: C07C 33/12, C07C 49/21, C07C 47/225, A61K 7/46

(54) **HYDROXY- OR OXO-SUBSTITUTED ALKYL CYCLOPENTENES**
HYDROXY- ODER OXOSUBSTITUIERTE ALKYLCYCLOPENTENE
ALKYLES CYCLOPENTENES A SUBSTITUTION HYDROXY OU OXO

(30) Priority: 10.06.1991 EP 91109493
(43) Date of publication of application: 26.05.1993
(73) Proprietor: GIVAUDAN-ROURE (INTERNATIONAL) S.A., CH-1214 Vernier (CH)
(72) Inventor: AUGER, Bernard, F-06530 Peymeinade (FR); GIRAUDI, Edouard, F-06550 La Roquette-sur-Siagne (FR)
(74) Representative: Cottong, Norbert A.
(86) International application number: EP9201243
(87) International publication number: WO9222518

(56) References cited:
- EP-A- 0 155 591
- DE-C- 3 441 902
- US-A- 4 149 020

## Description

The present invention relates to the field of perfumery. It concerns in particular a hydroxylated compound derived from α-campholene aldehyde, namely of the formula 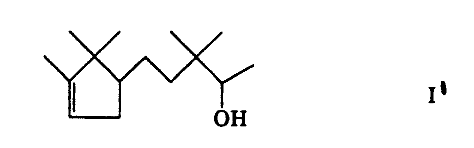
or 3,3-dimethyl-5-(2',2',3'-trimethyl-3'-cyclopenten-1'-yl)-pentan-2-ol, and its use as a fragrance.

The sandalwood note is highly sought after in perfumery. It is found in the natural essence of sandalwood, which is highly rated by the perfumer, not only because of its odorant character, but also owing to the fixative properties which it imparts in a number of compositions of various types.

The main compound which is responsible for the sandalwood odour in this essential oil is (-)-β-santalol, but two other components, (+)-α-santalol and lanceal, also contribute to the odour.

The prior art puts forward a large number of synthetic products whose odorant properties can be made ranked with those shown by the natural essential oil (E.J. Brunke and E. Klein "Chemistry of Sandalwood Fragrance" in Fragrance Chemistry, E.T. Theimer, publ. Academic Press, page 397, 1982).

In particular, great interest has been shown over a number of years in the derivatives of α-campholene aldehyde because of the sandalwood note which they frequently have, and which is much used and much appreciated in perfumery.

Thus, as early as 1969, Mühlstadt (GDR Patent 68936) reports that the compounds of the formula 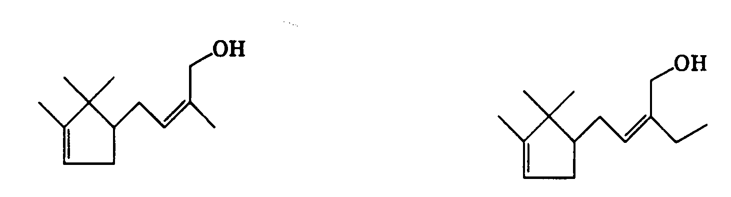
have an odour which is reminiscent of musk and sandalwood essence.

Subsequently, R.E. Naipawer (US-PS 4,052,341) has established that the compounds of the formula 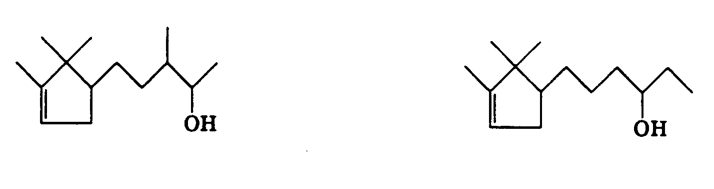
have a strong sandalwood odour.

Approximately at the same time, much effort was put into the derivatives of α-campholene aldehyde, giving rise to novel products, some of which were patented because of their significance in the field of perfumery; the following may be mentioned: 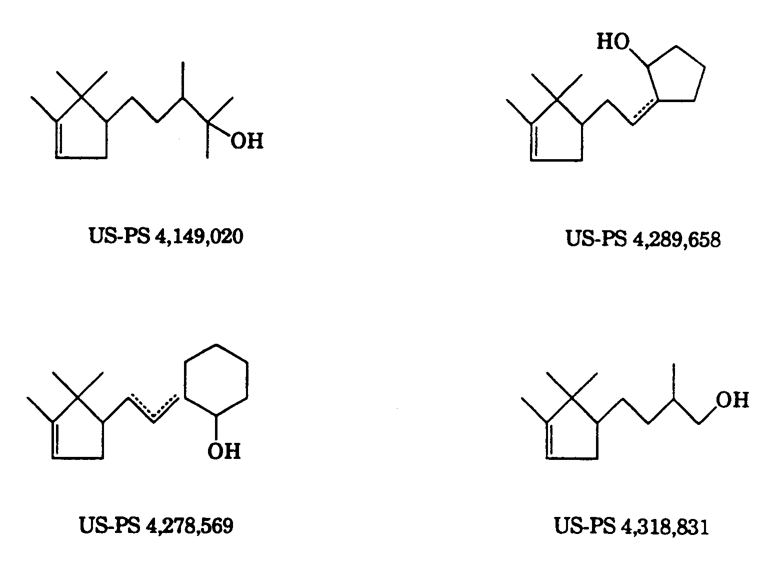
Subsequently, the introduction of a disubstituted double bond into the side chain containing 4 or 5 carbon atoms was found to be advantageous, allowing novel compounds to be introduced into this series. The following may be mentioned: 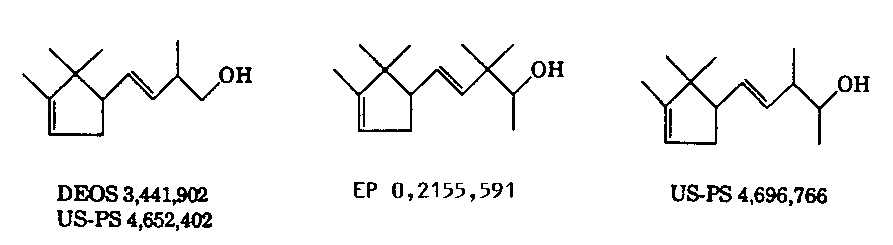
Several of these compounds having the campholene basic structure are commercially available products which are commonly used in perfumery. The following may be mentioned: 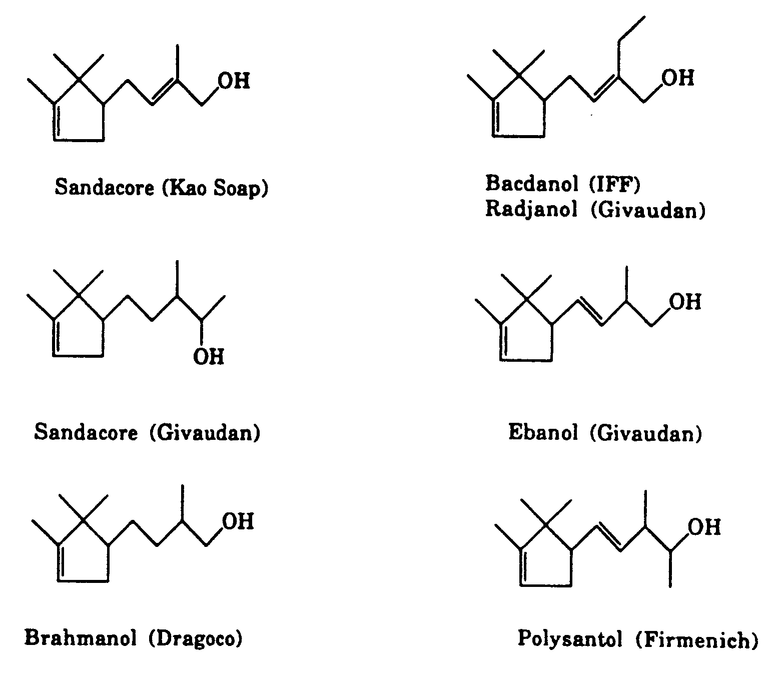
It thus seems that the number of synthetic compounds obtained from α-campholene aldehyde which have the sandalwood note is very large. The perfumer therefore has available a very wide range of compounds of this type, and one might think all the possible molecules have already been produced in this field, and that he already possesses all the odour notes in the sandalwood key.

In fact, this is not the case.

Indeed, careful examination of the structure of all molecules known to date which have the campholene basic structure has shown that those which comprise a saturated side chain containing 5 carbon atoms, is hydroxylated in the 2 position and disubstituted in the 3 position were original, and very particularly 3,3-dimethyl-5-(2',2',3'-trimethyl-3'-cyclopenten-1'-yl)pentan-2-ol.

Such a molecule can be prepared efficiently by using as starting material α-campholene aldehyde, which is obtained by extensively described methods by re-arrangement of epoxypinane (L.C. King and H. Farber, J. Org. Chem. 26, 326 (1961)). The enantiomerically pure forms of this compound can be obtained by using either (-)-α-epoxypinane or (+)-α-epoxypinane as starting compound (Beilstein E III 7 page 355) and can be used individually or as a mixture.

Two synthesis routes will be described which allow the novel compound I', to be prepared under satisfactory conditions: 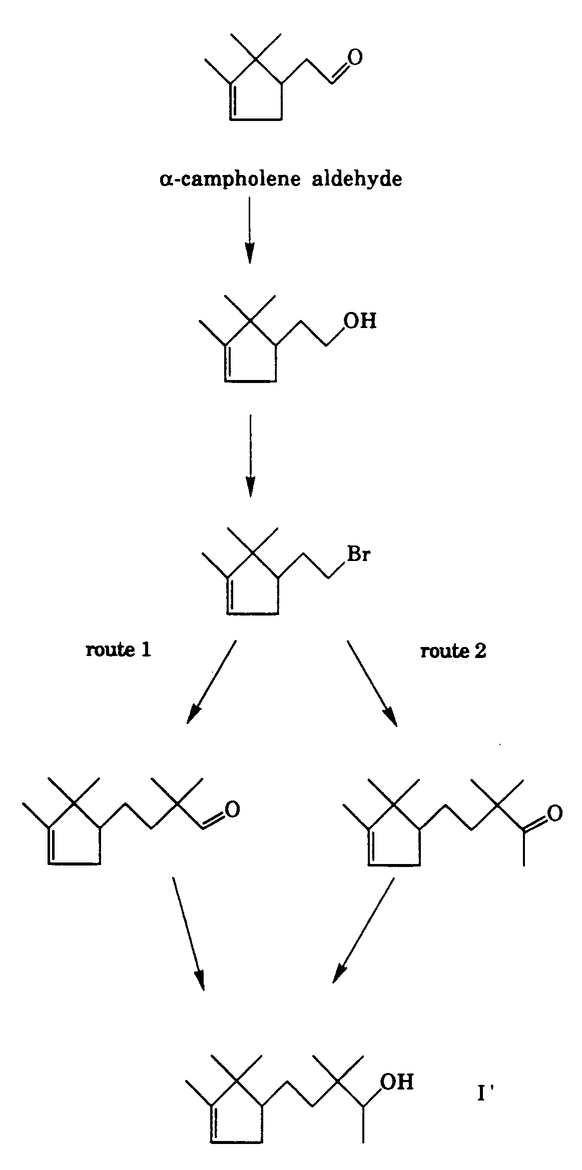
The starting material used in the these two synthesis routes is α-campholene aldehyde, which is first converted into an alcohol and then into the corresponding bromide following conventional and well-known methods (Examples 1, 2).

In the first case 2-(2',2',3'-trimethyl-3'-cyclopenten-1'-yl)-1-bromoethane is condensed with isobutyraldehyde, either directly in the presence of a base or indirectly via its enamine, following a general method which is extensively described in the literature (G. Stork et al., J. Am. Chem. Soc. 85, 207-222 (1963)). In this way, 2,2-dimethyl-4-(2',2',3'-trimethyl-3'-cyclopenten-1'-yl)butan-1-al is obtained (Example 3), a novel and original compound which is easily converted by reaction with an organometallic compound, for example a methyl organomagnesium compound or methyllithium - and expendiently using the standard conditions known for the Grignard reaction - into 3,3-dimethyl-5-(2',2',3'-trimethyl-3'-cyclopenten-1'-yl)-pentan-2-ol (Example 4).

In the second case, 2-(2',2',3'-trimethyl-3'-cyclopenten-1'-yl)-1-bromoethane is subjected to a condensation reaction with 3-methylbutan-2-one in the presence of a base such as an alkali metal hydride. In this way, 3,3-dimethyl-5-(2',2',3'-trimethyl-3'-cyclopenten-1'-yl)pentan-2-one (Example 5) is obtained, another novel and original compound which is easily converted by reaction with a reducing agent into the expected product I', (Example 6).

Suitable reducing agents are the usual reducing agents and methods respectively for ketones, i.e. complex hydrides, such as alkalimetal borohydrides and LiHH₄, Red-Al, or also the Meerwein-Panndoyl reagents, etc.

It was a great surprise to find that 3,3-dimethyl-5-(2',2',3'-trimethyl-3'-cyclopenten-1'-yl)-pentan-2-ol could develop useful notes which complement those having the sandalwood note which are already known and that it could therefore be used for perfuming various articles, bases and perfumes.

While it is true that the known compounds of the prior art can all impart a sandalwood note to the compositions, it cannot be denied that the resulting effects vary from one compound to the other and that not all impart the same complex and diverse effects of sandalwood essence at the same time. A person skilled in the art knows that the typical note of this natural compound results from a harmonious combination of various notes, mainly woody notes of the cedarwood or essence-of-guaiac type, but also balsamic, spicy, ambergris and animal notes, and also milky notes.

It is therefore not surprising that none of the products of the prior-art can, on its own, comprise all the nuances of odour and replace sandalwood essence.

In fact, it must be considered that each of them contributes to a greater or lesser extent to one or other of the particular olfactory characters which the natural product presents.

This may seem surprising in view of the great structural similarity which is shown by all known synthetic derivatives of α-campholene aldehyde. A study with the aim of defining the structural features which are common to these numerous molecules, all of which have the sandalwood note, has been carried out (R.E. Naipawer et al., Essential Oils, Ed. B.D. Mookherjee and C.J. Mussinam, Allured Publishing Corp., 105-133 (1981)) and very recently supplemented (M. Chastrette et al., Eur. J. Med. Chem 25, 433-440 (1990)).

In fact, the conclusions reached by the authors in this field remain rather sketchy and, cannot, actually explain the phenomenon of the exact correlation between the molecular structure of the products and their odour.

The uncertain character of any prediction regarding the structure/odour relationship is confirmed by the olfactory comparison of 3,3-dimethyl-5-(2',2',3'-trimethyl-3'-cyclopenten-1'-yl)pentan-2-ol with certain compounds of the prior art selected as being very similar in molecular structure.

In fact, the originality of the odour of compound I' of the present invention is striking, which, while having sandalwood tones which are reminiscent of the natural essence, presents a large number of original facets, which is why it has olfactory properties which are superior to those of the compounds of the prior art.

Thus, a person skilled in the art readily notices that 3,3-dimethyl-5-(2',2',3'-trimethyl-3'-cyclopenten-1'-yl)pentan-2-ol has volume from the beginning of its evaporation, unlike sandalore, polysantol and 2,2-dimethyl-4-(2',2',3'-trimethyl-3'-cyclopenten-1'-yl)-1-butanol, even though they are very similar in molecular structure.

Moreover, the tonality of each of these four products within the sandalwood note is different:
Compound I' is the only one to have the woody, chalky, hard edge of the natural sandalwood essence while sandalore is predominant in the sweet, milky tonality.

Polysantol has something of the woody edge of compound I' and also something of the milky edge of sandalore, but with tones which are floral and strange due to its "complex perfumery" edge to the natural sandalwood note.

The person skilled in the art also notices that 3,3-dimethyl-5-(2',2',3'-trimethyl-3'-cyclopenten-1'-yl)-pentan-2-ol, with its woody edge, contributes in a more precise manner to the approximation to the odour of the natural sandalwood essence, as has been the case with sandalore with its sweet, milky edge. In contrast, polysantol on its own appears more like a "fantasy" approach to the natural product.

Owing to this, the combination of compound I' with sandalore will contribute to a better olfactory approximation to the natural sandalwood essence than could polysantol or 2,2-dimethyl-4-(2',2',3'-trimethyl-cyclopenten-1'-yl)-1-butanol, which are nevertheless very similar in their molecular structure.

Compound I' therefore offers pronounced advantages for use in the field of perfumery.

In fact, this product, whose odour is reminiscent of the natural sandalwood odour, allows many harmonies with the whole range of natural and synthetic products which are universally known in perfumery and also with the head, middle and base components.

In harmony with natural products such as the peels of bergamot, lemon and orange, floral products such as rose, jasmine, iris, orange blossom, mimosa and violet, woody products such as oakmoss, patchouli and vetiver, and resins of the galbanum, incense, opopanax and elemi types.

In harmony with synthetic products:
- those with an alcohol functional group, such as geraniol, citronellol, linalool, phenylethyl alcohol, cinnamyl alcohol, cis-beta-hexenol,
- those with an aldehyde functional group, such as citral, cyclamen aldehyde, undecenal, methylnonylacetaldehyde, amylcinnamaldehyde, hexylcinnamaldehyde, hydroxycitronellal, lilial, popinal, lyral,
- with a ketone functional group such as alpha- and beta-ionone, irisantheme, methylionone, cis-jasmone, trans-jasmone, livescone, nectaryl,
- with an ester functional group, such as vetiveryl acetate, benzyl acetate, geranyl acetate, citronellyl acetate, linalyl acetate, cis-beta-hexenyl acetate, methyl dihydrojasmonate, benzyl salicylate, hexyl salicylate, amyl salicylate, allylamyl glycolate, geranyl formate, citronellyl formate, benzyl propionate, geranyl propionate, anatolyl propionate, centifolyl propionate, cyclohexyl crotonate, methyl-camomile, beta-cydrane,
- with a lactone functional group, such as gamma-undecalactone, hibiscolide,
- with a nitrile functional group, such as geranitrile, isogeranitrile, oranile,
- with a phenol functional group, such as eugenol, evernyl, orcinyl, 3-orcinyl,
and a large number of other components frequently used in perfumery, such as nitrated musks, indane-related musks, and pyrazines, etc.

Whereas the unmistakable, instantaneously recognisable character of I' as outlined above was already outlined "qualitatively", i.e. by simply bringing I as a 10% solution in ethanol onto smelling strips, such character could subsequently also been shown "quantitatively".

To this end, I' was brought to the extent of up to 30% into five different, experimental sandal wood bases.

A panel of three perfumers unanimously conceded that in each case the impact of I with regard to
volume and tonality and naturalness,
added value, tonality, originality,
development of a woody-flowery accord, volume and originality,
intensification of the accord,
equilibrium of the flowery and woody accord
was unquivocal.

The necessary comparison was effected with four parallel experiments, whereby the four sandalwood bases contained each - instead of I' - an equal amount of sandalwood essence, or
Bacdanol (A), or
Sandalore (B), or
Polysantol (C)
Sandal wood oil.

As can easily be gathered from the preceding paragraph A, B and C are structurally very closely related to I'.

The use percentage of compound I' can vary within a very wide range, from 0.1% in mass-market household products up to 25% in alcoholic extracts for designer perfumery, but these percentages are not limiting since the olfactory principle of this novel product allows great flexibility in use. Such values must not be interpreted in a restrictive manner since they depend on the nature of the product to be perfumed, on the nature of the other compounds which are present in the composition, and on the desired effect.

There are no restrictions at all with regard to the type of formulation or the intended use in finished products such as eau de cologne, toilet water, perfume spray, perfume, cream, shampoo, deodorant, soaps, washing powder, household cleaner, fabric softener, etc..

In conclusion, 3,3-dimethyl-5-(2',2',3'-trimethyl-3'-cyclopenten-1'-yl)pentan-2-ol can be incorporated in various use percentages into a large range of composition for any type of application used in the field of perfumery. With its olfactory note, it provides the necessary complement to the woody, dry tone of natural sandalwood.

To that end, three different formulation examples which use this product are added, demonstrating the general character of its use in perfumery and its olfactory significance and its important role at various dosage rates.

In Example 7, its use in a composition for a deodorant is illustrated, Example 8 relates to a composition for a fabric softener, and Example 9 to a composition for a designer perfume.

Examples 1 to 9 which are described below are intended to illustrate in a nonlimiting manner the possible preparations and applications of the compound I' in the field of perfumery.

### Example 1

### 2-(2',2',3'-trimethyl-3'-cyclopenten-1'-yl)ethan-1-ol

A 3-litre three-necked flask equipped with a mechanical stirrer, a condenser, a dropping funnel, a thermometer pocket and a nitrogen supply is charged with 53.2 g of NaBH₄ (1.4 mol) and 520 ml of absolute EtOH. The mixture is cooled to 0°C< t <5°C and, in the course of 2 hours, 152 g of α-campholene aldehyde are added (1 mol, GC purity 85%). The mixture is stirred for another 2 hours at 0°C< t <5°C, and then allowed to return to room temperature. The mixture is covered with 1600 ml of water. Countercurrent extraction is carried out with 2 300 ml of ethyl ether. After washing with H₂O, drying over MgSO₄ and removal of the solvent by distillation on a water bath, the crude reaction mixture is distilled under a high vacuum. This gives 132 g of 2-(2',2',3'-trimethyl-3'-cyclopenten-1'-yl)ethan-1-ol (yield = 86%).
B.p. = 94°C/1.3 mbar (1 mm Hg)
IR: 3334,3037,1462,1442,1379,1361,1055,799 cm⁻¹
¹H-NMR (200MHz; CDCl₃; δ ppm): 0.76 (3H,S); 0.97(3H,S);
1.60(3H,q,J = 2.5 Hz); 3.68 (2H,m); 5.22(1H,m).

### Example 2

### 2-(2',2',3'-trimethyl-3'-cyclopenten-1'-yl)bromoethane

A 500-ml flask equipped with a mechanical stirrer, a dropping funnel, a nitrogen supply and a thermometer pocket is charged with 22 ml of PBr₃ (0.23 mol = 63 g) and 66 ml of anhydrous CH₂Cl₂.
6.5 g of pyridine (0.08 mol) are then added at room temperature. The mixture is cooled to -5°C and a mixture containing 100 g of 2-(2',2',3'-trimethyl-3'-cyclopenten-1'-yl)ethan-1-ol (0.66 mol) and 6.5 g of pyridine (0.08 mol) is added over 15-20 minutes. Stirring at -5°C is continued for 1 hour and the mixture is then allowed to return to room temperature.

The precipitate formed is filtered off, the solvent is concentrated on a waterbath, and the crude reaction product is then distilled under a high vacuum. This gives 89 g of 2-(2',2',3'-trimethyl-3'-cyclopenten- 1'-yl)-1-bromoethane (yield = 62%).
B.p. = 86°C/1.3 mbar (1 mm Hg)
IR: 3020, 790 cm⁻¹
¹H-NMR (200 MHz; CDCl₃; δ ppm): 0.76 (3H,S); 0.98(3H,S);
1.60(3H,q,J = 2.5 Hz); 3.42(2H,m); 5.21(1H,m).

### Example 3

### 2,2-dimethyl-4-(2',2'-3'trimethyl-3'-cyclopenten-1'-yl)-butan-1-al

A 250-ml three-necked apparatus equipped with a mechanical stirrer, a reflux condenser, a dropping funnel and a thermometer pocket is placed under a nitrogen atmosphere is charged with 2.4 g of magnesium (0.1 at/g), and a mixture of 10.9 g of ethyl bromide (0.1 mol) in 12 ml of dry tetrahydrofuran is added. 15.3 g of isobutylenecyclohexylamine (0.1 mol), previously prepared by the method of R. Tiollais, Bull. Soc. Chim. France, 1947, page 715 and dissolved in 16 ml of dry THF are subsequently added to the tetrahydrofuran reflux. When the evolution of gas has ceased, the mixture is cooled to room temperature, and 21 g of 2-(2',2',3'-trimethyl-3'-cyclopenten-1'-yl)-1-bromoethane (0.1 mol) in 25 ml of dry THF are added over 15 minutes. The mixture is refluxed for 12 hours, hydrolysed (carefully) with 80 ml of 10% strength HCl, and stirred for another 3 hours while refluxing the THF.

After cooling, the mixture is subjected to countercurrent extraction with 3 250 ml of ethyl ether. The extract is washed with 250 ml of water saturated with sodium chloride, dried over magnesium sulphate, concentrated and distilled under a high vacuum. This gives 69 g of 2,2-dimethyl-4-(2',2',3'-trimethyl-3'-cyclopenten-1'-yl)butan-1-al (yield = 33%).
B.p. = 66-68°C/0.13 mbar (0.1 mm Hg)
¹H-NMR (200MHz; CDCl₃; δ ppm): 0.71(3H,S); 0.94(3H,S);
1.05(6H,S); 5.20(1H,S); 9.4(1H,S).

### Example 4

### 3,3-dimethyl-5-(2',2'-3'-trimethyl-3'-cyclopenten-1'-yl)-pentan-2-ol)

A 50-ml flask equipped with a magnetic stirrer, a reflux condenser, a dropping funnel and a thermometer pocket is placed under a nitrogen atmosphere and charged with 0.53 g of magnesium (0.022 at/g), and a mixture of 3 g of methyl iodide (0.021 mol) in 6 ml of anhydrous ethyl ether is added. The reaction is exothermal, and reflux conditions are maintained for 30 minutes after the end of the addition. The mixture is cooled to 0°C, and a solution of 4 g of 2,2-dimethyl-4-(2',2',3'-trimethyl-3'-cyclopenten-1'-yl)butan-1-al (0.019 mol) in 6 ml of anhydrous ethyl ether is run in at this temperature. Stirring is continued for 30 minutes at 0°C after the end of the addition, the mixture is allowed to return to room temperature, and stirring is continued for one hour at this temperature. The mixture is cooled to 0°C, and 20 ml of aqueous 10% strength ammonium chloride solution are run in. The mixture is transferred to a separating funnel, separated and extracted three times with 30 ml portions of ethyl ether. The combined organic phases are washed with water, dried over magnesium sulphate and concentrated. The crude reaction product is purified by flash chromatography on silica gel (5/95 Et₂O/PE). 4.2 g of 3,3-dimethyl-5-(2',2',3'-trimethyl-3'-cyclopenten-1'-yl)pentan-2-ol) are isolated (yield = 98%).
B.p. = 84°C/1.3 mbar (1 mm Hg)
IR: 3384,3036,1464,1381,1362,1095,911,800 cm⁻¹
¹H-NMR (200 MHz; CDCl₃; δ ppm): 0.75 (3H,S); 0.83(3H,S);
0.87(3H,S); 0.96(3H,S); 1.11(3H,d, J=7 Hz); 1.59(3H,q, J = 2.5 Hz); 3.56(1H,q, J = 7 Hz); 5.21(1H,m).
¹³C-NMR(50.3 MHz; CDCl₃): 12.64(CH₃)-17.10(CH₃)-19.80(CH₃)-22.55(CH₃)-22.6(CH₃)-23.92(CH₂)-26.01(CH₃)-35.87(CH₂)-37.41(C)-37.8(CH₂)-46.89(C)-51.2(CH)- 74.4(CH-OH)-121.7(=CH)-148.76(-C=C-).

### Example 5

A 500-ml three-necked apparatus equipped with a central mechanical stirrer, a reflux condenser and a thermometer pocket is placed under a nitrogen atmosphere and charged with 76 ml of anhydrous dimethylformamide, 18 g of 2-(2',2',3'-trimethyl-3'-cyclopenten-1'-yl)-1-bromoethane (0.08 mol) and 6.4 g of 3-methyl-2-butanone (0.075 mol). The mixture is cooled to 0°C and 7.16 g of 50% sodium hydride in Bayol (0.149 mol) [a paraffine oil] are added. Stirring is continued for 3 hours at 0°C, and the mixture is allowed to return to room temperature and is stirred for a further 15 hours at room temperature. 160 ml of pentane are added, the mixture is cooled to 0°C, and 160 ml of water are added slowly. The mixture is transferred to a separating funnel, separated and extracted three times with 160 ml portions of pentane. The combined organic phases are washed with water, dried over magnesium sulphate and concentrated on a Rotavapor apparatus. The crude reaction product is purified by a flash pass through on a silica column (eluent 5/95 Et₂O/PE).

6 g of 3,3-dimethyl-5-(2',2',3'-trimethyl-3'-cyclopenten-1'-yl)pentan-2-one are isolated (yield = 36%), having a purity of 95.5% according to GC analysis.
B.p. = 98°C/1.3 mbar (1 mm Hg)
IR: 3036,1707,1670,1358,799 cm⁻¹
1H-NMR (200 MHz; CDCl₃; δ ppm): 0.71(3H,S); 0.94(3H,S);
1.10(6H,S); 1.59(3H,q,J = 2.5 Hz); 2.10 (3H,S); 5.20 (1H,m).

### Example 6

A 250-ml three-necked apparatus equipped with a central mechanical stirrer, a reflux condenser, a thermometer pocket and a dropping funnel is placed under a nitrogen atmosphere and charged with 50 ml of anhydrous ethyl ether and 2 g of lithium aluminium hydride (0.052 mol). The mixture is cooled, and a solution of 6 g of 3,3-dimethyl-5-(2',2',3'-trimethyl-3'-cyclopenten-1'-yl)-pentan-2-one (0.027 mol) in 50 mol of anhydrous ethyl ether is run in dropwise with stirring. When the addition has ended, the mixture is allowed to return to room temperature, and stirring is continued for 3 hours.

The mixture is cooled to -5°C, and 2 ml of water and then 2 ml of 15% strength aqueous sodium bicarbonate solution and 6 ml of water are added. The mixture is filtered on a glass sinter, the filter cake is washed with ethyl ether, and the filtrate is concentrated on a Rotavapor apparatus. The crude reaction product is purified by a flash pass through a silica column (eluant 5/95 Et₂O/PE).

5.7 g of 3,3-dimethyl-5-(2',2',3'-trimethyl-3'-cyclopenten-1'-yl)pentan-2-ol) are isolated (yield = 94%), whose GC purity is 98% and whose physical characteristics are identical with those of the product obtained in Example 4.

### Example 7

| FRAGRANCE COMPOSITION (suitable for a deodorant) | | |
|---|---|---|
| ROSE OXIDE | 1 | --- (signifies equal parts per weight) |
| TRIPLAL IFF (4-formyl-2,6-dimethylcyclohexene) | 2 | --- |
| PEACH (undecalactone) | 3 | --- |
| EUGENOL | 5 | --- |
| SPIRAMBRENE (2',2'-dimethylspiro[carane-2,5'-(m)dioxane]) | 5 | --- |
| ALLYL AMYL GLYCOLATE | 6 | --- |
| CASCARILLA ECO ESS(ENCE) | 11 | --- |
| ARTEMESIA ESS | 12 | --- |
| BRAZIL ROSEWOOD | 25 | --- |
| ARTIFICIAL ESSENCE OF VERBENA | 35 | --- |
| BENZYL ACETATE | 60 | --- |
| MAGNOLIONE (3-acetonyl-2-pentylcyclopentanone) | 60 | --- |
| ISO E SUPER IFF (2-acetyl-tetramethyloctahydronaphthalenes) | 70 | --- |
| LINALYL ACETATE | 95 | --- |
| ARTIFICIAL ESSENCE OF BERGAMOTE | 95 | --- |
| BENZYL SALICYLATE | 100 | --- |
| HIBISCOLIDE (dioxo-2,7-cycloheptadecanone) | 115 | --- |
| IRISANTHEME (α-iso-methylionone) | 120 | --- |
| LILIAL (p-tert-butyl-α-methyldihydrocinnamaldehyde) | 120 | --- |
| DIPROPYLENE GLYCOL | 10 | 60 |
| 3,3-dimethyl-5-(2'2',3'-trimethyl-3'-cyclopenten-1'-yl)pentan-2-ol | 50 | --- |
| | 1 0̅0̅0̅ | 1̅ 0̅0̅0 |

3,3-dimethyl-5-(2',2',3'-trimethyl-3'-cyclopenten-1'-yl)pentan-2-ol imparts a great deal of sophistication to this composition and heightens the freshness character while giving it greater volume and persistence, by combining with this impression of sea-spray freshness, a certain warm bottom note.

This new substance has an effect both on the top and bottom notes of this composition by developing its very natural edge.

### Example 8

| FRAGRANCE COMPOSITION (for a fabric softener) | | |
|---|---|---|
| ALDEHYDE C.10 (decanal) | 5 | --- |
| ALDEHYDE C.9 (nonanal) | 5 | --- |
| ALDEHYDE C.12 MNA (2-methylundecanal) | 10 | --- |
| ALDEHYDE C.8 (octanal) | 15 | --- |
| HIBISCOLIDE (dioxa-2,7-cycloheptadecanone) | 20 | --- |
| METHYL GRAPEFRUIT (1,1-dimethoxy-2,2,5-trimethyl-4-hexene) | 20 | --- |
| SYNTHETIC GALBANUM RESINOID | 40 | --- |
| ROSEMARY ECO ESS | 40 | --- |
| VERTOFIX COEUR | 40 | --- |
| POPINAL 235 (methyl-4-cyclohexene-3-ylidene-1)-4-pentanal) | 50 | --- |
| BERGAMOT ECO ESS | 70 | --- |
| XYLENE MUSK | 80 | --- |
| HEXYLCINNAMALDEHYDE | 100 | --- |
| BRAZIL ORANGE ESS | 160 | --- |
| LEMON SUPER ECO ESS | 250 | --- |
| DIPROPYLENE GLYCOL | 85 | 95 |
| 3,3-dimethyl-5-(2',2'-3'-trimethyl-3'-cyclopenten-1'-yl)pentan-2-ol | 10 | --- |
| | 1 0̅0̅0̅ | 1̅ 0̅00 |

The addition of 1% of 3,3-dimethyl-5-(2',2',3'-trimethyl-3'-cyclopenten-1'-yl)pentan-2-ol increases the volume and persistence of the fresh citrus peel notes. The combination is more rich and homogeneous, and the "fresh, clean" impression is developed in the presence of the new substance.

### Example 9

| FRAGRANCE COMPOSITION (for designer perfume) | | |
|---|---|---|
| ALDEHYDE C.18 (nonalactone, Prunolide) | 5 | --- |
| ALPHA-DAMASCONE | 5 | --- |
| ALLYL AMYL GLYCOLATE | 10 | --- |
| TANGERINE ESS | 10 | --- |
| ARTIFICIAL ESSENCE OF CIVET | 10 | --- |
| NECTARYL (p-1-menthen-9-yl)-2-cyclopentanone | 20 | --- |
| ARTIFICIAL ESSENCE OF GALBANUM | 30 | --- |
| EUGENOL | 40 | --- |
| PATCHOULI ESS | 40 | --- |
| SPIRAMBRENE (2',2'-dimethylspiro[carane-2,5'-(m)dioxane]) | 50 | --- |
| PHIXIA (hydroxycitronellal) | 60 | --- |
| ABSOLUTE WOOD OAKMOSS | 70 | --- |
| VETIVER ESS | 80 | --- |
| GERANIUM ESS | 100 | --- |
| HIBISCOLIDE (dioxa-2,7-cyclohepta-decanone) | 100 | --- |
| BENZYL ACETATE | 100 | --- |
| ABSOLUTE JASMINE | 150 | --- |
| ROSEWOOD ESSENCE | 150 | --- |
| ARTIFICIAL ESSENCE OF ROSE ABS | 170 | --- |
| HEXYLCINNAMALDEHYDE | 200 | --- |
| VANILLIN 100% | 300 | --- |
| COUMARIN | 300 | --- |
| YLANG ESS | 300 | --- |
| IRISANTHEME (α-iso-methylionone) | 1500 | --- |
| MAGNOLIONE (3-acetonyl-2-pentylcyclopentanone) | 1500 | --- |
| ARTIFICIAL ESSENCE OF BERGAMOT | 1800 | --- |
| DIPROPYLENE GLYCOL | 500 | 2900 |
| 3,3-dimethyl-5-(2',2',3'-trimethyl-3'-cyclopenten-1'-yl)pentan-2-ol | 2400 | --- |
| | 10 0̅0̅0̅ | 1̅0̅ 0̅00 |

Volume, richness and warmth are imparted to this floral, fruity, green, woody harmony with a classical cypress note in which the excess of the sandalwood note due to the 3,3-dimethyl-5-(2',2'-3'-trimethyl-3'-cyclopenten-1'-yl)pentan-2-ol directs the warm tone towards a floral oriental harmony. The harmony imparted by the 3,3-dimethyl-5-(2',2'-3'-trimethyl-3'-cyclopenten-1'-yl)pentan-2-ol also works well with the floral peel of the top note and the sweet woody bottom note.

## Claims (Claims for the following Contracting State(s): CH, DE, FR, GB, IT, LI, NL)

1. A compound of formula 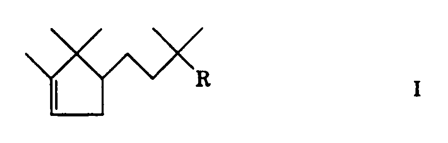 wherein R is the radical -CH(OH)CH₃ or -C(O)R¹ and R¹ is hydrogen or methyl.

2. 3,3-Dimethyl-5-(2',2',3'-trimethyl-3'-cyclopenten-1'-yl)pentan-2-ol.

3. 2,2-Dimethyl-4-(2',2',3'-trimethyl-3'-cyclopenten-1'-yl)butan-1-al.

4. 3,3-Dimethyl-5-(2',2',3'-trimethyl-3'-cyclopenten-1'-yl)pentan-2-one.

5. Process for the preparation of 3,3-dimethyl-5-(2',2',3'-trimethyl-3'-cyclopenten-1'-yl)pentan-2-ol, characterised in that a compound of the formula 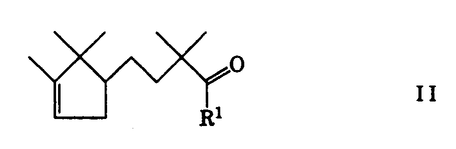 in which R¹ is hydrogen or methyl, is reduced, the reduction being carried out with an organometallic compound when R¹ is hydrogen.

6. Odorant compositions, characterised in that they contain 3,3-dimethyl-5-(2',2',3'-trimethyl-3'-cyclopenten-1'-yl)pentan-2-ol.

7. Use of 3,3-dimethyl-5-(2',2',3'-trimethyl-3'-cyclopenten-1'-yl)pentan-2-ol as a perfuming agent.

## Claims (Claims for the following Contracting State(s): ES)

1. Process for the preparation of 3,3-dimethyl-5-(2',2',3'-trimethyl-3'-cyclopenten-1'-yl)pentan-2-ol, characterised in that a compound of the formula 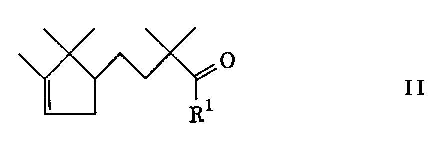 in which R¹ is hydrogen or methyl,
is reduced, the reduction being carried out with an organometallic compound when R¹ is hydrogen.

2. Odorant compositions, characterised in that they contain 3,3-dimethyl-5-(2',2',3'-trimethyl-3'-cyclopenten-1'-yl)pentan-2-ol.

3. Use of 3,3-dimethyl-5-(2',2',3'-trimethyl-3'-cyclopenten-1'-yl)pentan-2-ol as a perfuming agent.

## Patentansprüche (Patentansprüche für folgende(n) Vertragsstaat(en): CH, DE, FR, GB, IT, LI, NL)

1. Eine Verbindung der Formel 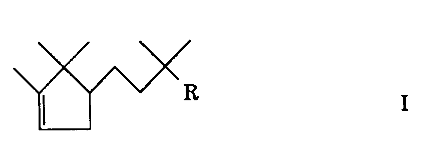 worin R das Radikal -CH(OH)CH₃ oder -C(O)R¹, und R¹ Wasserstoff oder Methyl darstellen.

2. 3,3-Dimethyl-5-(2',2',3'-trimethyl-3'-cyclopenten-1'-yl)pentan-2-ol.

3. 2,2-Dimethyl-4-(2',2',3'-trimethyl-3'-cyclopenten-1'-yl)butan-1-al.

4. 3,3-Dimethyl-5-(2',2',3'-trimethyl-3'-cyclopenten-1'-yl)pentan-2-on.

5. Verfahren zur Herstellung von 3,3-Dimethyl-5-(2',2',3'-trimethyl-3'-cylopenten-1'-yl)pentan-2-ol, dadurch gekennzeichnet, dass man eine Verbindung der Formel 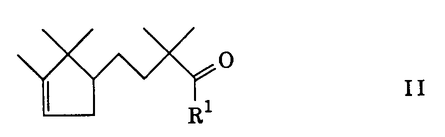 worin R¹ Wasserstoff oder Methyl ist,
reduziert, wobei die Reduktion im Falle von R¹ = Wasserstoff mit einer organometallischen Verbindung durchgeführt wird.

6. Riechstoffkompositionen, gekennzeichnet durch einen Gehalt an 3,3-Dimethyl-5-(2',2',3'-trimethyl-3'-cyclopenten-1'-yl)pentan-2-ol.

7. Verwendung von 3,3-Dimethyl-5-(2',2',3'-trimethyl-3'-cyclopenten-1'-yl)pentan-2-ol als Parfümierungsmittel.

## Patentansprüche (Patentansprüche für folgende(n) Vertragsstaat(en): ES)

1. Verfahren zur Herstellung von 3,3-Dimethyl-5-(2',2',3'-trimethyl-3'-cylopenten-1'-yl)pentan-2-ol, dadurch gekennzeichnet, dass man eine Verbindung der Formel 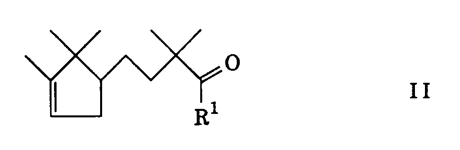 worin R¹ Wasserstoff oder Methyl ist,
reduziert, wobei die Reduktion im Falle von R¹ = Wasserstoff mit einer organometallischen Verbindung durchgeführt wird.

2. Riechstoffkompositionen, gekennzeichnet durch einen Gehalt an 3,3-Dimethyl-5-(2',2',3'-trimethyl-3'-cyclopenten-1'-yl)pentan-2-ol.

3. Verwendung von 3,3-Dimethyl-5-(2',2',3'-trimethyl-3'-cyclopenten-1'-yl)pentan-2-ol als Parfümierungsmittel.

## Revendications (Revendications pour l'(les) Etat(s) contractant(s) suivant(s): CH, DE, FR, GB, IT, LI, NL)

1. Composé de formule 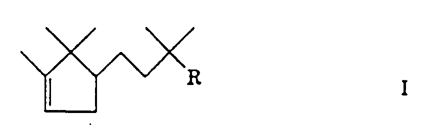 dans laquelle R représente le groupe -CH(OH)CH₃ ou -C(O)R¹ et R¹ représente un atome d'hydrogène ou le groupe méthyle.

2. 3,3-diméthyl-5-(2',2',3'-triméthyl-3'-cyclopenten-1'-yl)-pentan-2-ol.

3. 2,2-diméthyl-4-(2',2',3'-triméthyl-3'-cyclopenten-1'-yl)-butan-1-al.

4. 3,3-diméthyl-5-(2',2',3'-triméthyl-3'-cyclopenten-1'-yl)-pentan-2-one.

5. Procédé pour la préparation du 3,3-diméthyl-5-(2',2',3'-triméthyl-3'-cyclopenten-1'-yl)-pentan-2-ol, caractérisé en ce que l'on réduit un composé de formule 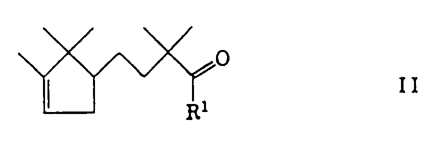 dans laquelle R¹ représente un atome d'hydrogène ou le groupe méthyle, la réduction étant réalisée avec un composé organométallique lorsque R¹ représente un atome d'hydrogène.

6. Compositions odorantes, caractérisées en ce qu'elles contiennent le 3,3-diméthyl-5-(2',2',3'-triméthyl-3'-cyclopenten-1'-yl)-pentan-2-ol.

7. Utilisation du 3,3-diméthyl-5-(2',2',3'-triméthyl-3'-cyclopenten-1'-yl)-pentan-2-ol, à titre d'agent parfumant.

## Revendications (Revendications pour l'(les) Etat(s) contractant(s) suivant(s): ES)

1. Procédé pour la préparation du 3,3-diméthyl-5-(2'_{,}2',3'-triméthyl-3'-cyclopenten-1'-yl)-pentan-2-ol, caractérisé en ce que l'on réduit un composé de formule 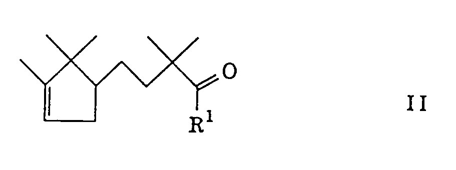 dans laquelle R¹ représente un atome d'hydrogène ou le groupe méthyle, la réduction étant réalisée avec un composé organométallique lorsque R¹ représente un atome d'hydrogène.

2. Compositions odorantes, caractérisées en ce qu'elles contiennent le 3,3-diméthyl-5-(2',2',3'-triméthyl-3'-cyclopenten-1'-yl)-pentan-2-ol.

3. Utilisation du 3,3-diméthyl-5-(2'_{,}2',3'-triméthyl-3'-cyclopenten-1'-yl)-pentan-2-ol, à titre d'agent parfumant.
